(19)

Europäisches
Patentamt
European
Patent Office
Office européen
des brevets

(11) **EP 3 785 617 A1**

(12) **EUROPEAN PATENT APPLICATION**

(43) Date of publication:
03.03.2021 Bulletin 2021/09

(51) Int Cl.:
A61B 5/021 (2006.01)     A61B 5/022 (2006.01)
A61B 5/1495 (2006.01)     A61B 5/00 (2006.01)

(21) Application number: 19193511.3

(22) Date of filing: 26.08.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• DERKX, Rene Martinus Maria
  5656 AE Eindhoven (NL)
• BRESCH, Erik
  5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **METHOD AND DEVICE FOR CALIBRATING BLOOD PRESSURE MEASUREMENTS BASED ON MEASURED PULSE ARRIVAL TIME**

(57)     A patient-specific calibration method is for deriving a calibration or relation between measured pulse arrival time values for a patient and arterial blood pressure of the patient. The calibration comprises acquiring at least one blood pressure measurement using a blood pressure measurement device which applies a varying pressure to an artery, and further acquiring one or more pulse arrival time measurements, each at a different value of applied pressure. One or more calibration coefficients are derived based on a model which relates PAT to blood pressure via a non-linear function of blood pressure and the applied pressure, the function incorporating the coefficients. The coefficients can be derived using this model function, based on substitution of the relevant measurements, or based on a curve fit procedure for example.

FIG. 2

EP 3 785 617 A1

**Description**

FIELD OF THE INVENTION

This invention relates to determining blood pressure measurements based on a measured pulse arrival time, and in particular to the calibration of such measurements.

BACKGROUND OF THE INVENTION

Blood pressure may be measured non-invasively by using a blood pressure measurement device, such as a cuff-based measurement device. Here, slowly varying external pressures are applied to the artery via a cuff placed on e.g. the brachial artery, and blood pulsations are measured through the cuff or via an auscultary measurement underneath the cuff. This is often used for spot-check measurements for example.

Other non-invasive but continuous measurement techniques are also possible, such as the vascular unloading technique. Here a finger-clamp is used to apply a counter pressure to the finger artery wall (as high as the blood pressure itself), to thereby maximally unload the finger artery wall. An optical measurement of the arterial volume in the finger is made and a feedback system implemented comprising dynamically adjusting the externally applied finger-cuff pressure such that the transmural pressure is minimized and the artery is maximally unloaded.

Alternatively to these non-invasive measurements, it is possible to estimate blood pressure continuously and without a cuff, via one or more surrogate measurements.

By way of example, it is known to estimate blood pressure continuously using the time-difference between the electrical activation of the heart and the arrival of the blood pressure wave at a further arterial location downstream from the heart, such as at an extremity such as the finger. This time-difference is known in the art as the pulse arrival time (PAT) and is known to relate very well to the blood pressure. Although the use of PAT to estimate blood pressure is known, the technique remains uncommon, and is not well established in clinical practice.

In some approaches, PAT can be measured via use of ECG (electrocardiography) and PPG (plethysmography) methods. ECG can be used to detect the onset of the QRS complex as the start of the electrical activation of the heart and the PPG can be used to detect the start of the pulse wave at the downstream location via optical measurements of the blood volume. Both measurements are standard measurements in clinical practice, for e.g. the intensive care unit of a hospital.

The time-averaged blood pressure (BP) is determined by cardiac output (CO) and total peripheral resistance (TPR), as represented by the formula: BP = CO × TPR. Cardiac output (CO) is defined as the amount of blood pumped per minute [Liters/min] and is hence affected by two factors, the heart rate (HR) [beats/min] and the stroke volume (SV), where the stroke volume is the volume of blood pumped from the left ventricle of the heart into the aorta with each beat [L/beat]. These factors are related to the cardiac output via the simple formula CO = HR × SV. Hence, for the blood pressure the following relation can be derived:

$$BP = \underbrace{HR \times SV}_{CO} \times TPR.$$

From this formula, it can be understood that the blood pressure increases when either the cardiac output (the amount of blood that the heart transports) is higher or when the total periphery resistance is higher (or both).

In reality, the blood pressure is not stable over time and dynamically varies between diastole and systole blood pressure within each heart cycle. Furthermore, the arterial system is complex and the elasticity and diameters of the arteries are variable throughout the arterial system. Although the time-averaged blood pressure decreases towards the extremities of the arterial system, the systolic blood pressure increases with greater proximity to the body peripheries, because the blood vessels are smaller at the peripheries. For example, it is known that the blood pressure at the brachial artery of the arm can be tens of mmHg lower compared to the blood pressure at the finger.

Furthermore, it is also known that it is not always easy to correlate these two blood pressures (as measured at the brachial artery, and as measured at the finger), because the hand and fingers have lots of smooth-muscles that can increase or decrease the vessel diameters very quickly over time. This is called vasoconstriction or vasodilation .

Changes in the pulse arrival time (PAT) will typically negatively correlate with the blood pressure changes. If the blood pressure increases, the blood vessels will become less compliant (i.e. stiffer) and as a consequence the time-delay between two arterial sites will be smaller (i.e. the PAT will be smaller).

In order to use PAT as a surrogate measure for the blood pressure estimation, it is necessary to relate or calibrate the two to one another, so that a certain measured PAT value can be related to a certain corresponding estimated blood pressure value.

However, it is known that this relation can change over-time and is subject dependent, especially when the cardiac-output (CO) is changed and/or when the smooth-muscle activation is changed. Known approaches to calibrating the surrogate PAT measurements and the blood pressure remain flawed, and frequently produce inaccurate and unreliable blood pressure measurements. Improving reliability and accuracy of blood pressure estimates, based on PAT measurements would therefore be of advantage.

SUMMARY OF THE INVENTION

The invention is defined by the claims.

According to examples in accordance with an aspect of the invention, there is provided a method of determining calibration values for use in deriving an estimated patient blood pressure, based on measured pulse arrival time (PAT), the method comprising:

obtaining one or more PAT values representative of a time difference between the start of a pre-ejection period of a heart cycle and the arrival of a corresponding pulse wave at a pre-determined first point on the body, as a result of blood flow from the heart to the first point through an arterial path of length, L;

obtaining from a blood pressure measurement device sensor data representative of at least one measured arterial blood pressure value, $P_0$, measured along a length portion $L_c$ of the arterial path, located between the heart and the pre-defined first point, wherein the blood pressure measurement device is configured to obtain blood pressure measurements through applying a varying external pressure, $P_{ext}$, across said length portion of the arterial path, $L_c$, wherein the one or more PAT values each correspond to PAT obtained at a different applied pressure, $P_{ext}$;

calculating, based on the one or more PAT values, and the corresponding $P_{ext}$ values, and on $P_0$, one or more coefficients, $m$, which relate a pulse wave velocity (PWV) along one or more sections of the arterial path with transmural pressure ($P_0$-$P_{ext}$) along the one or more sections, by modeling the one or more PAT values as a non-linear function of $P_{ext}$,

wherein the non-linear function takes the form:

$$\mathrm{PAT}(P_{ext}) = (\mathrm{L}\text{-}\mathrm{L}_c)\mathbf{f}(P_0, m) + \mathrm{L}_c\mathbf{f}(P_0\text{-}P_{ext}, m) + \mathrm{PEP} \qquad (1)$$

where $\mathbf{f}(x, m)$ is an inverse function of x of the form $\mathbf{f}(x, m) \propto 1/(mx + n)$, where n is a constant, and where PEP is a time duration of a pre-ejection period of the heart.

A calibration procedure is thus provided allowing for one or more calibration values (in the form of calibration coefficients) to be derived which will allow for measured PAT values to be related to blood pressure values for a particular patient. The calibration procedure may be patient specific therefore.

The calibration procedure is based on taking one or more reference or baseline arterial blood pressure measurements of the patient, using a dedicated blood pressure measurement device (e.g. a traditional oscillometric measurement via a blood pressure cuff), and also acquiring one or more PAT values for the patient concurrently. The PAT values are obtained at different values of applied external pressure $P_{ext}$. The one or more PAT values and the blood pressure measurement(s) may then be related to one another via a pre-defined model or relationship in the form of a non-linear function (defined above). This may be done by a curve fit procedure where multiple PAT values are acquired, or by simple algebraic calculation where there is only one PAT value.

The modelled relationship (1) includes a separate additive term corresponding to the time duration of the pre-ejection period (PEP) of the heart. PEP is a term of the art, and is the time period measured from the electrical activation of the heart (indicated for instance by the QRS complex in an ECG signal) until the ejection of the blood from the heart in the aortic artery. The pulse arrival time (PAT) is related to the PEP by: PAT = PEP + PTT, where PTT is the so-called pulse-transit time: the time between ejection of the blood into the aorta until the arrival of the pulse-wave at the downstream measurement location (the 'first point on the body' mentioned above).

The PEP period may typically take in the order of 100 milliseconds for example. The inventors have found that the PEP period can vary by a non-trivial amount from patient to patient. The inventors have found that by including the PEP period as a separate additive term in the model equation (1), more accurate blood pressure estimates can be derived, in particular for example compared to an approach in which the PEP term were to be incorporated into the other terms of the equation (which relate to the transit-time of the pressure-wave through the vessel). Such incorporation leads to an incorrect identification of the PWV sensitivity coefficients 'm' and the baseline PWV coefficient 'n' in the model equation (1).

The model equation includes two further additive terms which model the time duration of the transit of the blood pressure wave through different sections of the vascular path: the portion, $L_c$, to which an external pressure is being applied by the measurement device, and the remaining portion, $L-L_c$, to which no external pressure is being applied.

Each of the terms model the transit through the respective portion based on a function f of the transmural pressure across that portion of the vascular path, which function incorporates the calibration coefficients $m$. The function (denominator) models velocity of the pulse wave through the respective portion of the vascular path (pulse wave velocity), i.e. $(mx + n)$ represents pulse wave velocity through the respective portion of the vascular path. In this way, the coefficients $m$ relate pulse wave velocity to transmural pressure in the artery along the two portions of the arterial path. A transit time is recovered from this by the presence in each term of the length of the respective portion of the arterial path.

The constant $n$ may in some examples relate to a reference or baseline pulse wave velocity, meaning a pulse wave velocity in the absence of any applied external pressure, i.e. where $P_{ext} = 0$.

In advantageous examples, the non-linear function (1) may take the form:

$$PAT(P_{ext}) = \frac{L - L_c}{mP_0 + n} + \frac{L_c}{m(P_0 - P_{ext}) + n} + PEP \tag{2}$$

In advantageous examples, the constant $n$ may be taken to be:

$$n = \frac{L}{PAT_0 - PEP} - mP_0 \tag{3}$$

where $PAT_0$ is a reference or baseline pulse arrival time value, measured in the absence of any applied external pressure to the artery, i.e. where $P_{ext} = 0$. $PAT_0$ may therefore be understood as a reference or baseline value for pulse arrival time.

In advantageous examples, deriving the one or more coefficients, $m$, may be based on evaluating the expression:

$$m = P_{ext}^{-1}\left(\frac{L}{PAT_0 - PEP} - \frac{L_c}{(PAT(P_{ext}) - PEP) - (PAT_0 - PEP)\frac{(L - L_c)}{L}}\right) \tag{4}$$

As mentioned above, $PAT_0$ is a reference or baseline pulse arrival time value measured in the absence of any externally applied pressure to the artery, i.e. where $P_{ext} = 0$.

One coefficient may be derived for each of the obtained one or more PAT values. Additionally or alternatively, a single general or overall coefficient may be derived, based on the average of the coefficients corresponding to each of the measured PAT values, the average coefficient taking the form

$$m = \frac{1}{N}\sum_{i=0}^{N-1} P_{ext,i}^{-1}\left(\frac{L}{PAT_0 - PEP} - \frac{L_c}{(PAT(P_{ext,i}) - PEP) - (PAT_0 - PEP)\frac{(L - L_c)}{L}}\right) \tag{5}$$

As an alternative to algebraically calculating an average coefficient, a model fitting procedure may be used to fit a plurality of measured PAT and corresponding $P_{est}$ values to the PAT model equation (1) (or (2)).

According to one or more embodiments, the method may further comprise deriving based on the coefficients, $m$, one or more estimated blood pressure values based on newly measured PAT values, and based on the non-linear function (1) (or (2)). Thus the method may include the generation of estimated blood pressure values in addition to the initial calibration procedure described above.

The one or more estimated blood pressure values may advantageously be derived based on the equation:

$$P_{est} = \frac{L}{m}\left(\frac{1}{PAT - PEP} - \frac{1}{PAT_0 - PEP}\right) + P_0 \tag{6}$$

where $PAT_0$ is a reference or baseline pulse arrival time value measured without any externally applied pressure, i.e. where $P_{ext} = 0$.

This equation is based on the model equation (1) (or (2)) above, and can be derived from it. This will be explained further in the subsequent section.

In one or more embodiments, the method may include monitoring PAT values over time, and generating estimated blood pressure values based on the PAT values. Thus, PAT measurements may be obtained recurrently, for instance periodically, at regular intervals, and a blood pressure estimate derived for each PAT value.

In one or more advantageous embodiments, the method may additionally comprise monitoring the derived estimated blood pressure values for changes, and generating a control command for triggering a new blood pressure measurement by the blood pressure measurement device responsive to detecting a change in estimated blood pressure exceeding a pre-defined threshold. Thus a smart-trigger mechanism is implemented, whereby blood pressure measurements using the blood pressure measurement device are only triggered if a significant change in estimated blood pressure is detected. This avoids disturbing the patient repeatedly with blood pressure measurements that are uniformly triggered over time, for instance with a traditional oscillometric blood pressure measurement via a cuff. This is especially advantageous for example during periods when the patient is asleep, where the blood pressure measurement may wake the patient.

The one or more PAT values may be acquired based on use of a sensor device for optically and/or physically coupling to the body at said first point and configured to detect a pulse arrival at the first point, and preferably wherein the sensor device comprises a PPG sensor.

The first location (at which pulse arrival time is measured) may be the finger in advantageous examples. A finger PPG sensor may be used in some examples.

In one or more advantageous embodiments, the method may further comprise multiplying each of the one or more coefficients $m$ by a weighting $w_i$, the weighting representative of a quality metric of each of the one or more coefficients $m$, where this quality metric is determined based on analysis of signal features of a detection signal waveform of the sensor device, e.g. for each heart-cycle, and preferably based on comparison of said signal features with corresponding signal features of a pre-stored reference waveform.

In some examples, the analyzed signal feature is a signal strength.

Hence in some examples, the method may further comprise multiplying each of the one or more coefficients $m$ by a weighting $w_i$, the weighting representative of a relative signal strength of the sensor device in detecting the pulse arrival corresponding to the pulse arrival time $PAT(P_{ext})$.

It has been found by the inventors that one source of inaccuracy in blood pressure estimations can be PAT measurements where the upflank in the sensor signal used to detect pulse arrival is weak. Up-flank means for example the maximum gradient portion or maximum second-gradient portion of the sensor signal.

For example, PPG pulses can become dampened due to the attenuation of the pulse wave as it passes under the pressure-applying blood pressure measurement device. This can make detecting the signal upflank or peak or other fiducial point difficult. If this is not taken into account, it can mean that PAT measurements are obtained which are unreliable since they may be based on false positive detection of a pulse arrival or a false negative detection of pulse arrival.

Thus, in some examples the weighting coefficients $w_i$ for the PAT values with heavily attenuated PPG pulses (as detected for instance by a low signal amplitude compared with a baseline or reference sensor signal waveform) may be given a smaller (or zero) weight.

By applying weightings to each of the PAT values based on a quality metric of the pulse arrival signal, this source of inaccuracy can be reduced or eliminated.

According to these embodiments, optionally, in examples where a single overall coefficient is derived, as the average of the coefficients corresponding to each of the measured PAT values, the average coefficient may take the form

$$m = \frac{\sum_{i=0}^{N-1} w_i P_{ext,i}^{-1} \left( \dfrac{L}{PAT_0 - PEP} - \dfrac{L_c}{\left( PAT(P_{ext,i}) - PEP \right) - (PAT_0 - PEP)\dfrac{(L - L_c)}{L}} \right)}{\sum_{i=0}^{N-1} w_i}$$

where the values $w_i$ are the weightings referred to above.

In some examples, weightings, $w_i$, may be defined as follows

$$w_i = \begin{cases} 0 & when: \ S_{ext,i} < 0.1\, S_0 \ \ or: \ \ S_{ext,i} > 0.9\, S_0 \\ 1 & otherwise \end{cases}$$

where $S_{ext,i}$ is strength of the sensor device sensor detection signal for the i'th obtained PAT value when applying the external cuff pressure $P_{ext}$, and $S_0$ is a strength of a reference detection signal of the sensor device, measured in the absence of any applied external pressure.

Preferably, the blood pressure measurement device comprises an inflatable cuff.

Preferably the applied pressure, $P_{ext}$, referred to above is applied cuff pressure on the arterial length section $L_c$.

Preferably, the length section $L_c$ is the portion of the arterial path covered by the inflatable blood pressure cuff.

In examples, the method may comprise detecting a start time of the pre-ejection period based on an ECG signal obtained using an ECG sensor coupled to the patient. Start of the pre-ejection period may be indicated by an R-peak in the ECG signal for example.

In preferred examples, the blood pressure measurement(s) from the blood pressure measurement device may be representative of systolic blood pressure.

In some embodiments, the method may comprise controlling the blood pressure measurement device to acquire the blood pressure measurement and/or controlling the blood pressure measurement device to vary the applied pressure through a range of pressures.

In some embodiments, the method may comprise positioning the blood pressure measurement device on an arm of the patient for measuring the blood pressure, Po, (e.g. over a Brachial artery of the patient), and preferably such that said length section $L_c$ corresponds to a length section of the patient's arm.

Examples in accordance with a further aspect of the invention provide a controller unit configured to determine calibration values for use in deriving an estimated patient blood pressure measurement, based on measured pulse arrival time (PAT), the controller unit operably coupleable in use with a blood pressure measurement device, the controller configured to:

obtain one or more PAT values representative of a time difference between the start of a pre-ejection period of a heart cycle and the arrival of a corresponding blood pulse wave at a pre-determined first point on the body, as a result of blood flow from the heart to the first point through an arterial path of length, L;

receive from the blood pressure measurement device sensor data representative of at least one measured blood pressure value, $P_0$, measured along a length portion $L_c$ of the arterial path, located between the heart and the pre-defined first point, wherein the blood pressure measurement device is configured to obtain blood pressure measurements through applying a varying external pressure, $P_{ext}$, across said length portion of the arterial path, $L_c$, wherein the one or more PAT values each correspond to PAT obtained at a different applied pressure, $P_{ext}$;

calculate, based on the one or more PAT values, and the corresponding $P_{ext}$ values, and on $P_0$, one or more coefficients, m, which relate a pulse wave velocity (PWV) along one or more sections of the arterial path with transmural pressure ($P_0$-$P_{ext}$) by modeling the one or more PAT values as a non-linear function of $P_{ext}$,

wherein the non-linear function takes the form:

$$\text{PAT}(P_{ext}) = (\text{L-L}_c)\mathbf{f}(P_0, m) + \text{L}_c\mathbf{f}(P_0\text{-}P_{ext}, m) + \text{PEP}$$

where f(x, m) is an inverse function of x of the form f(x, m) $\propto$ 1/(mx + n), where n is a constant, and where PEP is a time duration of a pre-ejection period of the heart cycle.

Different options are possible for the obtaining of the PAT and values. The controller unit may receive the PAT values passively from a communicatively coupled sensor apparatus. Alternatively, the controller unit may control an operatively coupled sensor apparatus to obtain the PAT values.

The various options, examples and embodiments described above (or below) in relation to the method aspect of the invention may be applied or incorporated equally to the controller unit aspect described above.

Examples in accordance with a further aspect of the invention provide a system for determining calibration values for use in deriving an estimated patient blood pressure measurement, based on measured pulse arrival time (PAT), the system comprising:

a controller unit according to any example or embodiment described in this disclosure, or in accordance with any claim of this application; and
a blood pressure measurement device operatively coupled to the controller unit.

Preferably the blood pressure measurement device comprises an inflatable cuff. Preferably the applied pressure, $P_{ext}$, referred to above is applied cuff pressure on the arterial length section $L_c$. Preferably, the length section $L_c$ is the portion of the arterial path covered by the inflatable blood pressure cuff.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically depicts an example blood pressure measurement cuff in position on a patient's arm;
Fig. 2 schematically outlines an example electro-mechanical model of the arterial path from the heart to a downstream point for use in modelling a relationship between pulse arrival time and arterial blood pressure in accordance with one or more embodiments;
Fig. 3 shows results of an example calibration procedure in accordance with one or more embodiments;
Fig. 4 shows results of an example calibration procedure in accordance with one or more embodiments, wherein weightings are applied to calibration coefficients in accordance with a strength of a detection signal used to indicate pulse arrival at the arterially downstream point; and
Fig. 5 schematically shows in block diagram form an example system in accordance with one or more embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a patient-specific calibration method for deriving a calibration or relation between measured pulse arrival time values for a patient and arterial blood pressure of the patient. The calibration comprises acquiring at least one reference or baseline blood pressure measurement using a blood pressure measurement device which applies a varying pressure to an artery, and further acquiring one or more pulse arrival time measurements, each at a different value of applied pressure. One or more calibration coefficients are derived based on a model which relates PAT to blood pressure via a non-linear function of blood pressure and the applied pressure, the function incorporating the coefficients. The coefficients can be derived using this model function, based on substitution of the relevant measurements, or based on a curve fit procedure for example.

It is known that pulse arrival time (PAT) can be used as a surrogate measurement relating to the blood pressure of a patient. In case of a higher blood pressure, the arterial walls become less compliant (i.e. more stiff) and the pulse arrival time will typically therefore become shorter.

One possible application of PAT-based blood pressure estimates is for reducing the frequency of more intrusive direct blood pressure measurements, such as oscillometric blood pressure measurements, such as with a blood pressure measurement cuff. For example, one possible application is implementation of a smart-trigger system, wherein more direct blood-pressure measurements are made only responsive to detection of the surrogate-based estimated blood pressure measurements changing by some threshold amount or level.

For example, typically the triggering of oscillometric blood pressure measurements is done in hospitals at uniform time-intervals (e.g. every 15 minutes). This disturbs the patient which is inconvenient, especially when the patient is asleep. When the patient is asleep (e.g. in an intensive care unit), triggering with short time-intervals can deprive the patient of sleep due to cuff inflations (where external pressures are applied up to 200 mmHg) which are involved in the oscillometric blood-pressure measurements.

By using a surrogate blood pressure measurement instead, triggering of obtrusive oscillometric blood pressure measurements, such as with a cuff, can be adapted, e.g. with the smart trigger concept mentioned above. In this way, the frequency and total number of oscillometric measurements throughout the night will be reduced and only in response to detected changes of the blood pressure (e.g. exceeding some threshold) are measurements made via a more direct oscillometric method.

As discussed above, in order to use PAT as a surrogate measure of patient blood pressure, it is necessary to perform a calibration-step which allows for subsequent relating of measured PAT values to estimated blood pressure values. This calibration maybe performed based on use of an oscillometric blood pressure measurement, and measuring corresponding associated PAT values at the same time. For example, where a cuff-based measurement device is used, the calibration can be performed during the cuff inflation procedure (exploiting the cuff inflation originally intended for the oscillometric blood pressure reading).

Embodiments of the present invention provide a method of determining calibration values for use in deriving an estimated patient blood pressure measurement, based on measured pulse arrival time (PAT).

The method comprises obtaining one or more PAT measurements representative of a time difference between the start of a pre-ejection period of the heart and the arrival of a corresponding pulse wave at a pre-determined first point on the body, as a result of blood flow from the heart to the first point through an arterial path of length, L.

The method further comprises obtaining from a blood pressure measurement device sensor data representative of at least one measured blood pressure value, Po, measured along a length portion $L_c$ of the arterial path, located between the heart and the pre-defined first point, wherein the blood pressure measurement device is configured to obtain blood pressure measurements through applying a varying external pressure, $P_{ext}$, to said length portion of the arterial path, $L_c$, wherein the one or more PAT values each correspond to PAT obtained at a different applied pressure, $P_{ext}$. One reference PAT value, $PAT_0$, is obtained at an applied pressure of $P_{ext} = 0$. Further PAT values may also be obtained at other respective applied pressures. The at least one blood pressure measurement, $P_0$, may be obtained shortly after $PAT_0$ is obtained in examples.

The method further comprises calculating, based on the one or more PAT values, and the corresponding $P_{ext}$ values, and on $P_0$, one or more coefficients, *m*, which relate a pulse wave velocity (PWV) along one or more sections of the arterial path with transmural pressure ($P_0-P_{ext}$) by modeling the one or more PAT values as a non-linear function of $P_{ext}$.

In particular, the non-linear function takes the form:

$$PAT(P_{ext}) = (L-L_c)\mathbf{f}(P_0, m) + L_c\mathbf{f}(P_0-P_{ext}, m) + PEP$$

where f(x, m) is an inverse function of x of the form f(x, m) ∝ 1/(mx + n), where n is a constant, and where PEP is a time duration of a pre-ejection period of the heart.

The coefficients *m* effectively relate to a sensitivity of pulse wave velocity (PWV) within one or more sections of the arterial path with respect to transmural pressure in those sections of the arterial path.

Particular examples of the calibration method will now be described below which makes use of a blood pressure measurement device which comprises an inflatable cuff. However, this is not essential. The blood pressure measurement device may have a different construction, so long as it operates based on applying a varying pressure to an artery. This may be through a cuff or through a different pressure application approach.

Examples described herein may also make use of a blood pressure measurement device is fitted to a patient's arm to measure pressure in the brachial artery, and wherein pulse arrival time is measured at a finger of the patient. However, it is to be understood that these represent example locations only. The skilled person will recognize that other suitable measurement locations are possible for each of these parameters.

Certain examples herein also make use of a PPG sensor to sense pulse arrival at an arterial site on the body downstream from the blood pressure measurement device. A PPG sensor is just one example of a sensor which may be used to perform this measurement, and any other suitable sensor device may be used which can sense a pulse wave arrival. The measurement might even be performed manually for example.

Examples herein may also make use of an ECG sensor apparatus to sense a start time of the pre-ejection period. However, other means of sensing this time point may alternatively be used. In other examples, the time point may be estimated from other parameters. This will be explained in further detail to follow.

In accordance with one or more embodiments, the blood pressure measurement device may comprise an inflatable cuff configured to wrap around a patient's upper arm, covering a length portion of their brachial artery. The cuff inflation is varied to control a pressure $P_{ext}$ applied by the cuff to the arm and the underlying artery.

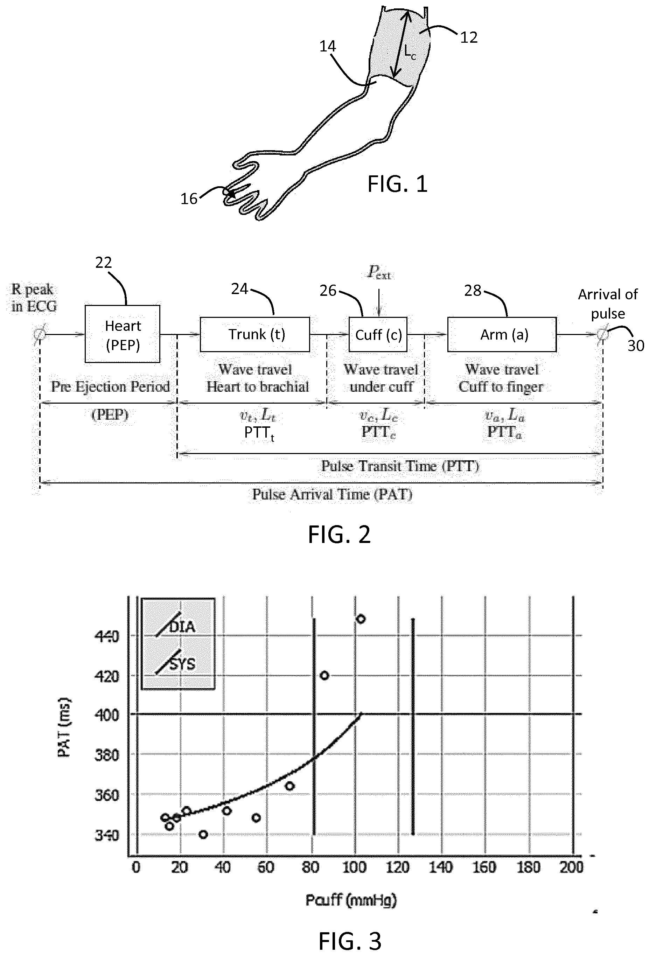

By way of illustration, Fig. 1 schematically depicts an example blood pressure measurement cuff 12 positioned on an upper arm 14 of a patient, for applying pressure to, and measuring blood pressure at, the brachial artery of the patient. The cuff covers a portion of the arm, and of the artery, of length $L_c$. This corresponds to the length of the cuff itself along the arm. Pulse arrival time may be measured downstream of the cuff 12, for example at the location of finger 16 of the patient. This may be done for instance using a PPG sensor fitted at the finger. Finger PPG sensors are well known in the art for example.

Measurement of blood pressure using a blood pressure measurement cuff is a well-known procedure, and the skilled person will be aware of means for implementing this. For example, the cuff can be gradually inflated from zero applied pressure to a maximum applied pressure, or can be gradually deflated from maximum applied pressure to zero applied pressure. A systolic and/or diastolic blood pressure measurement can be obtained based on monitoring variations in an internal pressure of the cuff inflation chamber due to pressure applied on the cuff by the underlying artery. Measures of this internal volume variation at different inflation levels (known in the field as "oscillations") can be processed using well-known algorithms to derive a measure of systolic and/or diastolic blood pressure.

As the cuff inflates (or deflates), one or more PAT values can be obtained at different applied pressures (different inflation levels of the cuff). Each obtained PAT value, and the corresponding $P_{ext}$ value, may be recorded, for instance in a memory.

Model coefficients are derived based on these one or more recorded PAT values, the corresponding $P_{ext}$ values, and on the at least one measured blood pressure value, Po. These model coefficients are derived based on a modelled relationship between PAT and blood pressure. The coefficients can be understood as correlation coefficients since, at a high level, they define how blood pressure is correlated with PAT, via defining how transmural pressure (which incorporates blood pressure) relates to pulse wave velocity (which is directly related to PAT via arterial length, L).

The coefficients themselves may relate pulse arrival time to pulse wave velocity along one or more sections of the arterial path from the heart to the downstream PAT arrival point, via terms representing blood pressure and applied pressure.

An example model for use in accordance with one or more embodiments will now be outlined in detail. The model will first be introduced in general terms, before describing its use within an example calibration procedure according to embodiments of the invention.

The model is based a multi-compartment model which considers the arterial path between the heart and the point at which the blood pulse arrival time is measured (referred to herein as the first point or downstream point) as comprising multiple sections or compartments. This multi-compartment model is schematically illustrated in Fig. 2.

The model encompasses four blocks or sections. One block 22 represents the electro-mechanical relation of the heart and the three subsequent blocks 24, 26, 28 represent successive sections of the arterial path extending from the heart to the downstream point 30 at which arrival of the pulse is measured. The second block 24 represents the section of the arterial path between the heart and the blood pressure measurement cuff. This section will be referred to as the trunk (t).The third block 26 represents the section of the arterial path which the cuff overlies and to which it applies an external pressure, $P_{ext}$. The fourth block 28 represents the section of the arterial path (along the arm) between the cuff and the downstream point 30 at which pulse arrival is measured.

The total length, $L$, of the arterial path between the heart and the lungs is given by the sum of the lengths of the each of the three sections 24, 26, 28, with the trunk section 24 having length $L_t$, the cuff section have length $L_c$, and the arm section 28 having length $L_a$.

The pulse wave velocity (PWV) along each of the sections 24, 26, 28 of the arterial path is also indicated in Fig. 2, with the velocity along the trunk section denoted $v_t$, the velocity along the cuff section denoted by $v_c$, and the velocity along the arm section denoted by $v_a$.

Blood pulse waves are generated by the heart after triggering via the sinus node, this causing contraction of the heart and ejection of the blood into the aorta. This ejection of the blood in the aortic artery generates a pressure wave through the blood. The electro-mechanical delay-time between the triggering and the ejection of the blood is called the pre-ejection-period (PEP). This period for example typically takes roughly 100 milliseconds (ms).

Next, the blood pressure waves travel (at roughly 5-10 m/s, depending on the elasticity and diameter of the specific artery) from the aortic artery to the downstream point 30 at which the pulse wave arrival is measured. In this example, this will be taken to be a point on the finger 30, but this represents just one example.

The time duration of the travel of the blood pressure wave from the heart (aortic artery) to the downstream point 30 is known as the pulse transit time (PTT). This travelling of this wave may typically take for example roughly 200-300 ms. The sum of the PTT and PEP is known as the pulse-arrival-time (PAT). This typically takes for example roughly 300-400 ms.

The calibration procedure is based on the generation by the cuff-inflation of an external pressure, $P_{ext}$, on the section of the artery (e.g. brachial artery), $L_c$, underlying the cuff. This can be understood as artificially increasing an effective compliance of the artery (known as 'vascular unloading' of the artery). As a result of this larger compliance, the pulse wave under the cuff will travel more slowly. This thereby increases the total PTT, and thus the total PAT. The model is based on the assumption that this artificial increasing of the compliance, and the consequent impact on the PTT and PAT is similar to the compliance change which occurs in response to a true blood pressure change. Thus the varying pressure of the blood pressure measurement device simulates changes in blood pressure, with consequent effect on the PAT measured, thereby allowing calibration or relation between the two to be derived.

With this calibration procedure, it is possible to identify a non-linear function that has to be applied to new PAT measurements in order to compute corresponding blood pressure.

The arterial blood pressure in compartment or section x (x being an element of the group of compartments {t,c,a}) will be denoted $P_x$.

During the calibration procedure, the applied pressure $P_{ext}$ of the blood pressure measurement device is increased from a minimum starting pressure to a maximum final pressure, i.e. the cuff is inflated from a starting pressure to a final pressure. From the oscillations captured by the cuff over the course of the cuff inflation at different moments of applied pressure $P_{ext}$, a reference or baseline blood pressure measurement, $P_0$ is derived.

Assuming that $P_x=P_0$ for all compartments 24, 26, 28, the travel speed (pulse wave velocity), $V_x$, of the pressure-wave in each of the compartments x may be given by:

$$v_x = \frac{L_x}{\mathrm{PTT}_x} \approx m\,P_x + n, \qquad\qquad (2.1)$$

where $m$ and $n$ are model-parameters and $P_x$ is the blood pressure in the compartment x.

The sensitivity parameter $m$ has units of [m/ms/mmHg], since it describes the relation between the pulse-wave speed change [m/ms] and the blood pressure change [mmHg]. This parameter, $m$, provides the sensitivity or calibration coefficient(s) which are derived by the calibration method. These may be referred to herein as the calibration coefficient(s), $m$.

The offset parameter $n$ has units [m/ms] to calibrate for the baseline pulse-wave speed, meaning the pulse wave velocity in the absence of applied external pressure, i.e. where $P_{ext} = 0$.

The average travel speed via all three arterial compartments $t, c$ and $a$ maybe understood as:

$$v = \frac{L}{\mathrm{PTT}} = \frac{L}{\mathrm{PAT} - \mathrm{PEP}} \approx m\,P_0 + n, \qquad\qquad (2.2)$$

with: $L = L_t + L_c + L_a$ and where PTT is the pulse transit time from the heart to the finger (or other pulse arrival measurement point), and PAT is the measured pulse arrival time.

By way of example, the PAT may be measured using an ECG sensor apparatus and a PPG sensor. The ECG sensing apparatus allows detection of the time point of the start of the pre-ejection period. This can be detected as corresponding to an R-peak in the ECG signal. Pulse arrival at the finger (or other downstream point) can be detected with a PPG sensor located at the pulse arrival point 30, based on detection of an upflank in the PPG signal. A finger PPG sensor may be used for example. The PAT can be taken as the difference between the detected start of the PEP period and the detected pulse arrival.

As discussed above, the parameter Po is a measured reference blood pressure value used for relating PAT to arterial blood pressure. In practice, this blood pressure value may be measured for example via inflation of the measurement cuff and measurement of the oscillations in the cuff during inflation. The sequence of the oscillations (and the applied pressure to the artery by the cuff at the moments of the oscillations) are utilized to measure the blood pressure values (systolic blood-pressure = $P_0$, diastolic blood-pressure and mean-arterial blood pressure). This is well-known procedure in the field. This blood pressure measurement is then assumed to correspond to the blood-pressure $P_0$ inside the artery in the absence of any externally applied pressure. This means that it can be assumed that the artery in reality experiences the arterial pressure $P_0$ just before and just after the cuff inflation.

The parameter PEP may be considered as a fixed parameter, e.g. estimated at roughly 100 ms.

The PEP parameter is known to be related to the diastolic blood pressure. Thus the PEP parameter may also be estimated based on:

$$\mathrm{PEP} \approx 16.405 + 0.858 \cdot P_{\mathrm{dia}}, \qquad\qquad (2.3)$$

where $P_{dia}$ is the diastolic blood pressure as obtained via the standard oscillometric measurement, e.g. using the measurement cuff.

For the derivations hereafter and the results shown, an estimated PEP value will be used of approximately 100 ms. This value is for purposes of illustration of the concept of the invention only, and is not to be considered limiting to the scope of the invention. Other PEP values may be used without effect on the advantages of the invention.

In performing the calibration method according to embodiments of the invention, several measurements must be taken.

The first measurement is $PAT_0 = PAT(P_{ext} = 0)$ which is the baseline pulse-arrival-time that can be measured prior to the cuff inflation, when there is no applied pressure by the cuff: $P_{ext} = 0$. Using the above-described model, the following equation maybe derived:

$$PAT(P_{ext} = 0) \triangleq PAT_0 = PEP + \sum_{x \in \{t,c,a\}} \frac{L_x}{mP_0 + n} = PEP + \frac{L}{mP_0 + n} \qquad\qquad (2.4)$$

where the parameter $P_0$ is the baseline or reference blood pressure value discussed above (in preferred examples, the systolic blood pressure value, although mean arterial blood pressure may alternatively be used for example) obtained during the cuff inflation via a traditional oscillometric measurement. This oscillometric measurement may be obtained for example just after the measurement of the baseline pulse-arrival-time $PAT_0$, e.g. substantially concurrently with measurement of $PAT_0$

It is also know that during the cuff inflation the compliance of the artery in the cuff compartment 26 (i.e. in the section of the artery beneath the cuff), is artificially increased by reducing the transmural pressure $P_0$ - $P_{ext}$ along the section, $L_c$, of the arterial path beneath the cuff. From this assumption, it is possible, using the above-described model to derive a second equation:

$$PAT(P_{ext}) = PEP + \frac{L_c}{m(P_0 - P_{ext}) + n} + \sum_{x \in \{t,a\}} \frac{L_x}{mP_0 + n} \qquad (2.5)$$

$$= \frac{L - L_c}{mP_0 + n} + \frac{L_c}{m(P_0 - P_{ext}) + n} + PEP$$

The measurement-pair (PAT($P_{ext}$), $P_{ext}$), representing the measured PAT value at an applied pressure of $P_{ext}$, and the applied pressure itself are obtained (and stored) during the cuff-inflation. At least one (PAT($P_{ext}$), $P_{ext}$) measurement pair is obtained during the inflation. More than one (PAT($P_{ext}$), $P_{ext}$) measurement pair may be obtained, wherein each PAT measurement is obtained at a different respective $P_{ext}$ value.

The above provides PAT as a non-linear function of $P_{ext}$. The function includes three terms. One term represents the time duration of the pre-ejection period, PEP. This is included as a separate additive term in the equation. The other two terms represent a time duration for transit of the pressure wave through different portions of the arterial path. The term $\frac{L_c}{m(P_0 - P_{ext}) + n}$ represents the time duration for transit of the pressure wave through the section 26 of the arterial path underlying the cuff. The term $\frac{L - L_c}{mP_0 + n}$ represents the time duration of transit of the pressure wave through the remaining sections 24, 28 of the arterial path.

Each term $\frac{L_c}{m(P_0 - P_{ext}) + n}$ and $\frac{L - L_c}{mP_0 + n}$ comprises an inverse function f(x, m), of the form f(x, m) $\propto$ 1/(mx + n). Each represents more particularly an inverse function of the transmural pressure, $P_{tm}$ = $P_0$ - $P_{ext}$, along the portion of the arterial path to which the term relates. For example, the term $\frac{L_c}{m(P_0 - P_{ext}) + n}$ relating to the portion of the arterial path beneath the cuff is an inverse function of $P_0$ - $P_{ext}$ which presents the transmural pressure (net pressure) across the artery wall along the section being applied with pressure by the cuff (i.e. arterial pressure - cuff pressure). For the term $\frac{L - L_c}{mP_0 + n}$, likewise, this is an inverse function of $P_0$ which is the transmural pressure (net pressure) along the remaining portion of the arterial path, where there is no applied pressure, i.e. where transmural pressure equals arterial pressure.

More generally therefore, the relation between PAT($P_{ext}$) and arterial pressure may be represented by a non-linear function of $P_{ext}$, where the non-linear function takes the form:

$$PAT(P_{ext}) = (L-L_c)\mathbf{f}(P_0, m) + L_c\mathbf{f}(P_0\text{-}P_{ext}, m) + PEP \qquad (1)$$

where $\mathbf{f}$(x, m) is an inverse function of x of the form $\mathbf{f}$(x, m) $\propto$ 1/(mx + n), where m is a calibration or sensitivity coefficient which relates to the sensitivity of the pulse wave velocity (PWV) with respect to the transmural pressure ($P_0$-$P_{ext}$), n is a constant, and PEP is a time duration of a pre-ejection period of the heart.

Furthermore, the denominator of each term of equation (2.5) $\frac{L_c}{m(P_0 - P_{ext}) + n}$ and $\frac{L - L_c}{mP_0 + n}$ represents a pulse wave velocity of blood along the respective portion(s) of the arterial path to which the term relates. The calibration coefficient, m, thus relates arterial pressure (via transmural pressure) in the respective portion(s) of the arterial path with pulse wave velocity in the respective portion(s) of the arterial path. The constant $n$ represents an offset or baseline pulse wave velocity.

Both parameters $m$ and $n$ are in the denominator of the non-linear relationship between PAT and blood pressure. Hence, this model can be understood as a so-called 'inverse' model, since many methods in the art use a model that linearly relates PAT and blood pressure, whereas in the inverse model, the pulse-wave-velocity (PWV) and blood pressure are assumed to be linearly related.

As can be seen from Eq. 2.5 (and Eq. (1)), the modelled relationship between PAT and blood pressure includes a separate additive term corresponding to the time duration of the pre-ejection period (PEP) of the heart. The inventors have found that the PEP period can vary by a non-trivial amount over time. It also varies from patient to patient. The inventors have found that by including the PEP period as a separate additive term in the model equation (2.5) (or (1)), more accurate blood pressure estimates can be derived, in particular for example compared to an approach in which the PEP term were to be incorporated into the other terms of the equation (which relate to the transit-time of the pressure wave through the vessel). Such incorporation leads to an incorrect identification of the PWV sensitivity coefficients 'm' and the baseline PWV coefficient 'n' in the model equation (1) (and equation (2.5)).

For example, were the PEP duration to be incorporated into e.g. the baseline PWV coefficient, $n$, (which would simplify the equation) this would lead to inaccuracy in the PAT relationship. In particular, each of the terms of equation (2.5) (or Eq. 1) which include $n$ are scaled by a length value, representing the length of the respective portion of the arterial path to which the term corresponds. However, PEP does not scale with length (it is a fixed value for a given pulse), hence, its incorporation into $n$ would result in inaccuracy as a result.

Equation (2.4) maybe combined with equation (2.5) by first rearranging Eq. (2.4) to derive following equation for the constant $n$:

$$n = \frac{L}{\text{PAT}_0 - \text{PEP}} - m\,P_0, \qquad (2.6)$$

The non-linear function of equation (2.5) may be used to derive the coefficients $m$, based on at least one measured PAT value and corresponding $P_{ext}$ value, this being the value of the cuff applied pressure at the time of measuring the PAT value.

One approach to deriving the calibration coefficient(s) is algebraically, based on re-arranging the equations (2.6) and (2.5) above.

For example, by substituting Eq. (2.6) into Eq. (2.5), and eliminating the parameters $n$ and $P_0$, the following may thereby be yielded:

$$PAT(P_{ext}) = PEP + \underbrace{(PAT_0 - PEP)\left(\frac{L - L_c}{L}\right)}_{\text{offset}} + \underbrace{\frac{L_c}{\frac{L}{PAT_0 - PEP} - mP_{ext}}}_{1/x\,\text{curve}} \qquad (2.7)$$

As mentioned, for Eq. (2.7), it is necessary to have obtained at least one measurement-pair (PAT($P_{ext}$), $P_{ext}$) during the cuff inflation. With this at least one measurement-pair, it is possible to identify the unknown calibration parameter $m$.

When multiple PAT measurements are available, the calibration coefficient, $m$, may be derived by performing a curve fitting procedure, wherein the multiple PAT and corresponding $P_{ext}$ values are fitted to the function represented in Eq. (2.7). In particular, just the "1/x" term of Eq. (2.7), which includes the $m$ parameter may be used. A 'best-fit' of the multiple $P_{ext}$, PAT pairs to the 1/$x$ part maybe derived.

Deriving the one or more correlation coefficients may be done algebraically based on rearranging of Eq. (2.7). Eq. (2.7) maybe rearranged to yield the following equation

$$m = P_{ext}^{-1}\left(\frac{L}{PAT_0 - PEP} - \frac{L_c}{(PAT(P_{ext}) - PEP) - (PAT_0 - PEP)\frac{(L - L_c)}{L}}\right) \qquad (4)$$

One calibration coefficient, $m$, may be derived for each acquired PAT, $P_{ext}$ value pair.

Additionally or alternatively, a single general or overall coefficient may be derived, based on an average of the coefficients corresponding to each of the measured PAT values.

The computation of the average m of N measurement-pairs (PAT($P_{ext,i}$), $P_{ext,i}$) with $i = 0.. N - 1$ may be done via rearranging of Eq. (2.7) and linear averaging of the parameter m as follows:

$$m = \frac{1}{N} \sum_{i=0}^{N-1} P_{ext,i}^{-1} \left( \frac{L}{PAT_0 - PEP} - \frac{L_c}{\left(PAT(P_{ext,i}) - PEP\right) - (PAT_0 - PEP)\frac{(L - L_c)}{L}} \right) \quad (2.8)$$

This overall, or average, parameter m is a positive valued number that describes the average speed change (pulse wave velocity change) in [m/s] of the pulse-wave along the path from the heart to the downstream point at which the pulse arrival is measured (e.g. the finger) with respect to the transmural pressure ($P_0$-$P_{ext}$) in [mmHg].

According to one or more embodiments, the method may further comprise deriving based on the coefficients, m, one or more estimated blood pressure values based on newly measured PAT values, and based on the non-linear function of Eq. (2.5). Thus the method may include the generation of estimated or surrogate blood pressure values in addition to the initial calibration procedure described above.

To estimate the surrogate blood pressure value(s) $P_{est}$, at least one PAT value must be obtained. Together with the calibration coefficient m as identified in either equation (4) or (2.8), the baseline coefficient n and the estimated PEP parameter, it is possible to estimate the blood pressure by using Eq. (2.6) together with Eq. (2.2) to yield:

$$P = \frac{v - n}{m} = \frac{\frac{L}{PAT - PEP} - n}{m} \quad (2.9)$$

The parameter n is a baseline PWV value that may be computed in accordance with Eq. (2.6).

Using Eq. (2.6), it is possible to re-write Eq. (2.9) as:

$$P_{est} = \frac{L}{m} \left( \frac{1}{PAT - PEP} - \frac{1}{PAT_0 - PEP} \right) + P_0 \quad (2.10)$$

where $PAT_0$ is a baseline pulse arrival time value, measured in the absence of any externally applied pressure, i.e. where $P_{ext} = 0$. One or more estimated blood pressure values, $P_{est}$, may advantageously be derived based on equation (2.10). The average correlation coefficient, m, derived in Eq. (2.8) may be used in Eq. (2.10), or the single value may be used, as in Eq. (4).

Further to the above, according to one or more embodiments, there may be derived information related to change in blood pressure [mmHg] as a function of change in PAT [ms]. This can be derived for example based on computing the gradient of the estimated blood pressure in Eq. (2.10) with respect to the parameter PAT to obtain a sensitivity s [mmHg/ms]. This may be done based on computing the partial derivative with respect to PAT to yield:

$$\frac{\partial P_{sur}}{\partial PAT} \triangleq s = -\frac{L}{m(PAT - PEP)^2}. \quad (2.11)$$

By making the assumption that the PAT change with respect to. the baseline value of $PAT_0$ is only small, it is possible to derive the following approximation of the sensitivity factor, s:

$$s \approx -\frac{L}{m(PAT_0 - PEP)^2}. \quad (2.12)$$

In one or more advantageous embodiments, the method may additionally comprise monitoring the derived estimated blood pressure values for changes, and generating a control command for triggering a new blood pressure measurement by the blood pressure measurement device responsive to detecting a change in estimated blood pressure exceeding a pre-defined threshold. Thus a smart-trigger mechanism is implemented, whereby blood pressure measurements using the blood pressure measurement device are only triggered if a significant change in pressure is detected.

This avoids disturbing the patient repeatedly with manual blood pressure measurements, for instance with a blood pressure cuff. This is especially advantageous during periods when the patient is asleep, where the blood pressure measurement may wake them.

The one or more PAT values may be acquired based on use of a sensor device for optically and/or physically coupling to the body at said first point and configured to detect a pulse arrival at the first point, and preferably wherein the sensor device comprises a PPG sensor.

The first location (at which pulse arrival time is measured) may be the finger in advantageous examples. A finger PPG sensor may be used in some examples.

In one or more advantageous embodiments, the method may further comprise applying to each of the one or more coefficients $m$ a weighting $w_i$, the weighting representative of a quality metric of each of the one or more coefficients m, where this quality metric is determined based on one or more signal characteristics or features of a waveform of the sensor device signal for each received pulse wave.

The quality metric may be based for example on comparison of said signal features or characteristics with corresponding signal features or characteristics of a pre-stored reference waveform.

In one set of examples, the weighting may be representative of a relative strength of a detection signal used in detecting the pulse arrival corresponding to the pulse arrival time $PAT(P_{ext})$ compared to a signal strength of a reference waveform for the detection signal. Hence here, the quality metric is a signal strength.

Hence in some examples, the method may further comprise multiplying each of the one or more coefficients $m$ by a weighting $w_i$, the weighting representative of a relative signal strength of the sensor device signal in detecting the pulse arrival corresponding to the pulse arrival time $PAT(P_{ext})$, the signal strength relative to a baseline or reference waveform for the detection signal.

In particular, it has been found by the inventors one source of inaccuracy in blood pressure estimations can be that the obtained PAT measurements at high cuff pressures have poor reliability because the PPG signal used to detect pulse arrival is damped and hence the upflank (or other fiducial feature) in the PPG sensor signal, e.g. the maximum velocity or maximum acceleration of the PPG signal, used to detect the pulse arrival is weak.

The dampening of the PPG signal at high cuff pressures is caused because the inflated cuff will fully or partially occlude the artery underneath the cuff and will block the pulse wave through this arterial section.

Thus, in some examples the weighting coefficients $w_i$ for the PAT values with heavily attenuated PPG pulses (as detected for instance by a low signal amplitude compared with a baseline or reference sensor signal waveform) may be given a smaller (or zero) weight.

In further examples, it has also been found by the inventors that in the early phase of cuff inflation, the externally applied cuff pressure, $P_{ext}$, is so low that the PAT measurements are not yet significantly influenced by a change in pulse wave velocity induced by the $P_{ext}$ change. Including these PAT measurements would not be beneficial for the computation of the calibration coefficients 'm' since the difference between the baseline PATo value and the measured PAT is too small. Inclusion of these $m$ coefficient values may lead to at least a partial source of inaccuracy.

Hence, in some examples, the weighting coefficients $w_i$ for these poorly conditioned values of 'm' may be given a smaller (or zero) weight. These values may be detected for example based on comparison of the acquired PAT value used to compute given correlation coefficient $m$, with the baseline $PAT_0$ value. For example, where the difference between the two is below a predefined threshold, a low or zero weighting might be applied to the corresponding correlation coefficient $m$.

Thus, in some examples the weighting coefficients $w_i$ for the PAT values with heavily attenuated PPG pulses (as detected for instance by a low signal amplitude compared with a baseline or reference sensor signal waveform) may be given a smaller (or zero) weight.

It has also been found by the inventors that in the early phase of the cuff inflation, the externally applied cuff pressure is too low for the PAT measurements to yet be significantly influenced by a change in pulse wave velocity. Including these measurements may not be beneficial for the computation of the coefficient 'm', and may lead to some inaccuracy.

By way of one example, in examples where a single overall correlation coefficient, m, is derived, as the average of the coefficients corresponding to each of the measured PAT values, the average coefficient may take the form

$$m = \frac{\sum_{i=0}^{N-1} w_i P_{ext,i}^{-1} \left( \dfrac{L}{PAT_0 - PEP} - \dfrac{L_c}{PAT(P_{ext,i}) - PEP - (PAT_0 - PEP)\dfrac{(L - L_c)}{L}} \right)}{\sum_{i=0}^{N-1} w_i} \qquad (2.13)$$

This equation can be derived by modifying equation (2.8) for example.

In accordance with one set of examples, the weights $w_i$ may be determined as:

$$w_i = \begin{cases} 0 & when: \ S_{ext,i} < 0.1 \ S_0 \quad or: \quad S_{ext,i} > 0.9 \ S_0 \\ 1 & otherwise \end{cases} \qquad (2.14)$$

where $S_{ext,i}$ is strength of the sensor device sensor detection signal for the i'th obtained PAT value when applying the external cuff pressure $P_{ext}$, and $S_0$ is a strength of a reference detection signal of the sensor device, measured in the absence of any applied external pressure. The detection signal may be a PPG sensor signal for example.

Hence, in this example, the weighting is set to zero where the pulse wave detection signal strength is less than 10% of the strength of the reference detection signal. This hence excludes correlation coefficients, $m$, calculated for pulse waves having heavily attenuated signal strength. The weighting is also set to zero where the pulse wave detection signal strength is more than 90% of the reference detection signal. This may typically indicate that the detection signal corresponds to a pulse obtained at an early stage of cuff inflation (since attenuation is so low), and hence for which the PAT value is potentially too close to a reference $PAT_0$ value to obtain a useful $m$ coefficient result.

It will be understood that this represents just one example, and other example approaches to quantifying the weightings can alternatively be used. In particular, the example described in equation (2.14) uses binary values of the weighting $w_i$. However, in other examples, a continuous equation may be used which defines the weighting as a continuous parameter, varying continuously based on signal strength $S_{ext,\ i}$, of the detection signal.

As discussed above, embodiments of the invention are based on modeling the obtained PAT values as a non-linear function of externally applied pressure on the artery, $P_{ext}$, where the non-linear function takes the form $PAT(P_{ext})$ = $(L-L_c)\mathbf{f}(P_0, m) + L_c\mathbf{f}(P_0-P_{ext}, m) + PEP$, where $\mathbf{f}(x, m)$ is an inverse function of x.

In examples discussed above, a non-linear function in the form set out in equation (2) or equation (2.5) above for example has been discussed. However, other example non-linear functions are also possible.

For example, in the function of equation (2.5), the denominator of the inverse function f comprises a single-order polynomial, such that $f(x, m) \propto 1/(mx + n)$, where n is a constant. As an alternative, a higher order polynomial could be used. For example, a suitable non-linear equation might take the form

$$PAT(P_{ext}) = \frac{L - L_c}{kP_0{}^2 \ mP_0 + n} + \frac{L_c}{k(P_0 - P_{ext})^2 + m(P_0 - P_{ext}) + n} + PEP$$

where two correlation or sensitivity coefficients, $m$, $k$, are calculated for each obtained PAT value for instance.

In further alternatives, the denominator of the inverse function might take the form of an exponential function.

According to one or more embodiments, a calibration method according to any of the examples or embodiments described above may be implemented by a processor or by a computer. There may be provided a computer program product comprising code means configured, when executed on a processor, the processor being operatively coupled to a blood pressure measurement device and to a means for obtaining PAT measurements from a patient, to cause the processor to perform the calibration method according to any of the examples or embodiments described above. The code may further cause the processor to derive one or more estimated blood pressure values for the patient.

By way of example, example computer code (in the Python language) for implementing a calibration method according to one or more examples is set out below.

```
def calc_cal_weighted(Pcuff, PAT_ref, PAT, w, Pmap, Psys, Lcuff, L, PEP):
    """
    Description : Compute curve-fit for calibration
    Pre : Pcuff : array with cuff pressures [mmHg]
                PAT_ref : single PAT value for baseline [ms]
                PAT : array with PAT (Pulse Arrival Time) [ms]
                w : weights for curvefit
                Psys : Systolic BP value from oscillometric
                measurement [mmHg]
                Lcuff : Length of the cuff [m]
                L : Length of the heart to the PPG sensor [m]
                PEP : Pre ejection period [ms]
    Post : psys_from_pat_func : Function definition Psys = f(PAT)
                pat_from_pcuff_func : Function definition PAT = f(Pcuff)
      """ sensitivity : Calibrated sensitivity [mmHg/ms]
    Pcuff_weighted=np.multiply(Pcuff, w)
```

```
    pwv_to_sys_slope = np.dot(Pcuff_weighted, \
      np.multiply(w, (L/(PAT_ref-PEP) - Lcuff/((PAT-PEP) - (L-
  Lcuff)/L*(PAT ref-PEP)))) ) / \
      (np.dot(Pcuff_weighted, Pcuff_weighted))
    psys_from_pat_func = lambda pat: L/pwv_to_sys_slope*(1/(pat-PEP)-
  1/(PAT_ref-PEP)) + Psys
    pat_from_pcuff_func = lambda pcuff: PEP+Lcuff/(L/(PAT_ref-PEP)-
    pcuff*pwv_to_sys_slope) + \
      ((L-Lcuff)/L*(PAT_ref-PEP))
    sensitivity = (-L/pwv_to_sys_slope)/((PAT_ref-PEP)*(PAT_ref-PEP))
    return psys_from_pat_func, pat_from_pcuff_func, sensitivity
```

This code directly utilizes equation (2.13) set out above to derive the correlation coefficient, $m$, from which surrogate estimated blood pressure values may be derived. The resulting m coefficient is a weighted average of all the values of 'm' obtained during cuff inflation, as explained in relation to equation (2.13) (and (2.8)) above.

Here, the input-parameters for the calibration-function are:

- The (PAT, Pcuff) value pairs are given by (PAT($P_{ext}$), $P_{ext}$)
- The baseline blood pressure as given by $P_0$
- The baseline PAT as given by $PAT_0$
- The baseline PPG pulse strength as given by $S_0$
- The weights $w_i$, are computed as: w=PPG_strengths_buffer > 0.1 * S0 and PPG_strengths_buffer < 0.9 * S0 where PPG_strengths_buffer contains the strengths of the PPG values during the inflation
- The cuff-length, $L_c$, given by Lcuff
- The total arterial path-length L of the patient given by L
- An estimate of the pre-ejection time PEP, is given by PEP

By means of the output function, estimated (surrogate) blood pressure measurements may be derived (by using PAT measurements) based on:

```
 BP_est = psys_from_pat_func(1000.0 * PAT)
```

where PAT is the set of measured PAT values, in units of seconds, for which blood pressure (BP) estimates are to be derived, one estimated BP value for each measured PAT value. BP_est represents the derived estimated blood pressure value (the surrogate blood pressure value), in units of [mmHg]. The function psys_from_pat_func is a translation function between PAT and blood pressure, as computed by the overall calc_cal_weighted function. It is defined in the calc_cal_weighted function set out above as:

```
 psys_from_pat_func = lambda pat: L/pwv_to_sys_slope*(1/(pat-PEP)-
 1/(PAT_ref-PEP)) + Psys
```

The above code performs a weighted averaging procedure using equation (2.13), from which the calibration coefficients 'm' can be derived. The 'm' coefficient is computed by the variable 'pwv_to_psys_slope' in the Python function outlined above, which follows the form of equation (2.13) above. The psys_from_pat_func function mentioned above is the translation function (i.e. the non-linear function) that translates the obtained PAT values into a surrogate blood pressure estimate.

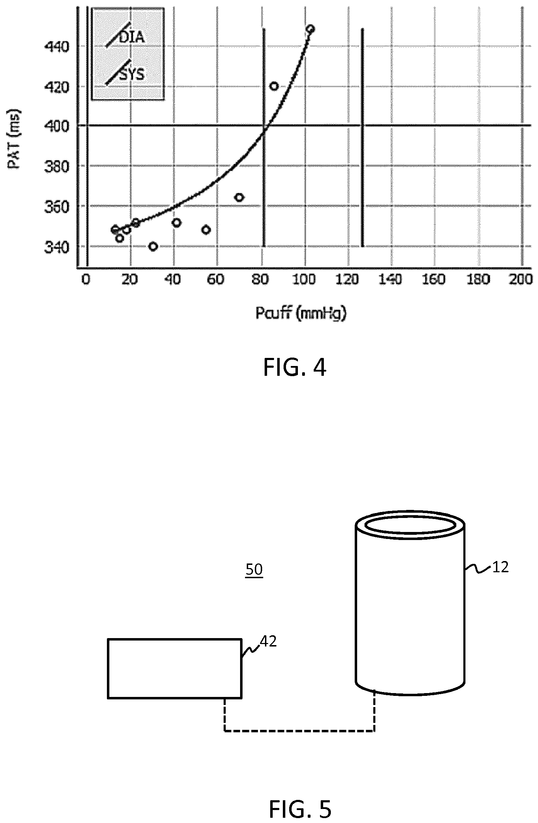

To illustrate the improvements provided by use of the weightings, the results of the curve fit of equation (2.5) using a non-weighted method via equation (2.8) and using a weighted method via equation (2.13) are shown respectively in Fig. 3 and Fig. 4.

It can be seen that the fit is much improved where weightings are used, by which is meant that the resulting surrogate blood pressure measurements for given PAT values obtained using $m$ coefficients derived using the weightings (equation 2.13) fit much more closely to the observed blood pressure measurements for those same PAT measurements than do $m$ coefficients obtained without weightings (equation 2.8). This is shown in the two graphs, which each relate to the same single subject, and correspond to results for a weighting-based method (left) and for a non-weighting based method (right).

The technique with weighting (via equation (2.13)) also shows an improved reproducibility in terms of the derived values of the $m$ coefficients, compared with the technique without use of the weightings (via equation (2.8)).

In some embodiments, the method may comprise controlling the blood pressure measurement device to acquire the blood pressure measurement and/or controlling the blood pressure measurement device to vary the applied pressure through a range of pressures.

In some embodiments, the method may comprise positioning the blood pressure measurement device on an arm of the patient for measuring the blood pressure, $P_0$, (e.g. over a brachial artery of the patient), and preferably such that said length section $L_c$ corresponds to a length section of the patient's arm.

Examples in accordance with a further aspect of the invention provide a controller unit configured to determine calibration values for use in deriving an estimated patient blood pressure measurement, based on measured pulse arrival time (PAT), the controller unit operably coupleable in use with a blood pressure measurement device.

The controller is configured to obtain one or more PAT values representative of a time difference between the start of a pre-ejection period of a heart cycle and the arrival of a corresponding blood pulse wave at a pre-determined first point on the body, as a result of blood flow from the heart to the first point through an arterial path of length, L.

The controller is further configured to receive from the blood pressure measurement device sensor data representative of at least one measured baseline blood pressure value, $P_0$, measured along a length portion $L_c$ of the arterial path, located between the heart and the pre-defined first point, wherein the blood pressure measurement device is configured to obtain blood pressure measurements through applying a varying external pressure, $P_{ext}$, across said length portion of the arterial path, $L_c$, wherein the one or more PAT values each correspond to PAT obtained at a different applied pressure, $P_{ext}$.

The controller is further configured to calculate, based on the one or more PAT values, and the corresponding $P_{ext}$ values, and on $P_0$, one or more coefficients, *m*, which relate a pulse wave velocity (PWV) along one or more sections of the arterial path with transmural pressure ($P_0$-$P_{ext}$) by modeling the one or more PAT values as a non-linear function of $P_{ext}$,

wherein the non-linear function takes the form:

$$PAT(P_{ext}) = (L-L_c)\mathbf{f}(P_0, m) + L_c\mathbf{f}(P_0\text{-}P_{ext}, m) + PEP$$

where f(x, m) is an inverse function of x of the form f(x, m) $\propto$ 1/(mx + n), where n is a constant, and where PEP is a time duration of a pre-ejection period of the heart cycle.

Different options are possible for the obtaining of the PAT values. The controller unit may receive the PAT values passively from a communicatively coupled sensor apparatus. Alternatively, the controller unit may control an operatively coupled sensor apparatus to obtain the PAT values.

The various options, examples and embodiments described above in relation to the method aspect of the invention may be applied or incorporated equally to the controller unit aspect described above.

Examples in accordance with a further aspect of the invention provide a system for determining calibration values for use in deriving an estimated patient blood pressure measurement, based on measured pulse arrival time (PAT).

An example system 50 in accordance with one or more embodiments is shown in Fig. 5. The system comprises a controller unit 42 according to any example or embodiment described in this disclosure, or in accordance with any claim of this application, and further comprises a blood pressure measurement device 12 operatively coupled to the controller unit. The blood pressure measurement device in the example shown in Fig. 5 is a cuff-based oscillometric blood pressure measurement device. The blood pressure measurement device may be connected with the controller unit 42 via a wired or wireless connection.

The controller unit may in some examples be further operatively coupled with means for acquiring pulse arrival time (PAT) measurements for a patient. This means may comprise for example an ECG sensing apparatus for sensing a start of the pre-ejection period of the patient's heart, and a PPG sensor for sensing the arrival of the corresponding pulse wave at a point arterially downstream from the heart. This represents just one example set of apparatus for making PAT measurements, and the skilled person will recognize other apparatus that might alternatively be used.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A method of determining calibration values for use in deriving an estimated patient blood pressure measurement, based on measured pulse arrival time (PAT), the method comprising:

   obtaining one or more PAT values representative of a time difference between the start of a pre-ejection period of a heart cycle and the arrival of a corresponding pulse wave at a pre-determined first point on the body, as a result of blood flow from the heart to the first point through an arterial path of length, L;
   obtaining from a blood pressure measurement device sensor data representative of at least one measured arterial blood pressure value, P0, measured along a length portion Lc of the arterial path, located between the heart and the pre-determined first point, wherein the blood pressure measurement device is configured to obtain blood pressure measurements through applying a time-varying external pressure, Pext, to said length portion of the arterial path, Lc, wherein the one or more PAT values each correspond to PAT obtained at a different applied pressure, Pext;
   calculating, based on the one or more PAT values, and the corresponding Pext values, and on P0, one or more coefficients, m, which relate a pulse wave velocity (PWV) along one or more sections of the arterial path with transmural pressure (P0-Pext) by modeling the one or more PAT values as a non-linear function of Pext, wherein the non-linear function takes the form:

   $$\mathrm{PAT(P_{ext})} = \mathrm{(L\text{-}L_c)}\mathbf{f}(\mathrm{P_0}, m) + \mathrm{L_c}\mathbf{f}(\mathrm{P_0\text{-}P_{ext}}, m) + \mathrm{PEP}$$

   where f(x, m) is an inverse function of x of the form f(x, m) $\propto$ 1/(mx + n), where n is a constant, and where PEP is a time duration of a pre-ejection period of the heart cycle.

2. The method of claim 1, wherein the non-linear function of claim 1 takes the form:

   $$PAT(P_{ext}) = \frac{L - L_c}{mP_0 + n} + \frac{L_c}{m(P_0 - P_{ext}) + n} + PEP$$

3. The method of claim 2, wherein the constant n is taken to be:

   $$n = \frac{L}{PAT_0 - PEP} - mP_0$$

   where $PAT_0$ is a measured pulse arrival time value for an applied pressure $P_{ext} = 0$.

4. The method of any of claims 1-3, wherein deriving the one or more coefficients, m, is based on evaluating the expression:

   $$m = P_{ext}^{-1}\left(\frac{L}{PAT_0 - PEP} - \frac{L_c}{(PAT(P_{ext}) - PEP) - (PAT_0 - PEP)\frac{(L - L_c)}{L}}\right)$$

   where PATo is a measured pulse arrival time value for an applied pressure $P_{ext} = 0$.

5. The method of any of claims 1-4, wherein one coefficient is derived for each acquired PAT value; and/or
   wherein a single coefficient is derived, as the average of the coefficients corresponding to each of the measured PAT values, the average coefficient taking the form

$$m = \frac{1}{N} \sum_{i=0}^{N-1} P_{ext,i}^{-1} \left( \frac{L}{PAT_0 - PEP} - \frac{L_c}{\left(PAT(P_{ext,i}) - PEP\right) - (PAT_0 - PEP)\frac{(L - L_c)}{L}} \right)$$

6. The method of any of claims 1-5, the method further comprising deriving based on the coefficients, $m$, one or more estimated blood pressure values based on newly measured PAT values, and based on the non-linear function of any of claims 1-3.

7. The method of claim 6, wherein the one or more estimated blood pressure values are derived based on the equation:

$$P_{est} = \frac{L}{m} \left( \frac{1}{PAT - PEP} - \frac{1}{PAT_0 - PEP} \right) + P_0$$

where $PAT_0$ is a measured pulse arrival time value for an applied pressure $P_{ext} = 0$.

8. The method of claim 6 or 7, wherein the method includes monitoring PAT values over time, and generating estimated blood pressure values based on the PAT values, and further comprising generating a control command for triggering a new blood pressure measurement by the blood pressure measurement device responsive to detecting a change in estimated blood pressure exceeding a pre-defined threshold.

9. The method of any of claims 1-8, wherein the one or more PAT values are acquired based on use of a sensor device for optically and/or physically coupling to the body at said first point and configured to detect a pulse arrival at the first point, and preferably wherein the sensor device comprises a PPG sensor.

10. The method of claim 9, further comprising multiplying each of the one or more coefficients $m$ by a weighting $w_i$, the weighting representative of a quality metric of each of the one or more coefficients m, where this quality metric is determined based on analysis of signal features of a detection signal waveform of the sensor device, and preferably based on comparison of said signal features with corresponding signal features of a pre-stored reference waveform.

11. The method of claim 10, wherein a single coefficient is derived, as the average of the coefficients corresponding to each of the measured PAT values, the average coefficient taking the form

$$m = \frac{\sum_{i=0}^{N-1} w_i P_{ext,i}^{-1} \left( \frac{L}{PAT_0 - PEP} - \frac{L_c}{\left(PAT(P_{ext,i}) - PEP\right) - (PAT_0 - PEP)\frac{(L - L_c)}{L}} \right)}{\sum_{i=0}^{N-1} w_i}$$

12. The method of any of claims 1-11, wherein the blood pressure measurement device comprises an inflatable cuff, and wherein the applied pressure, $P_{ext}$, is applied cuff pressure on the length portion $L_c$, and wherein the length portion, $L_c$, is the portion of the arterial path covered by the inflatable cuff.

13. A computer program product comprising code means configured, when executed on a processor, to cause the processor to perform the method of any of claims 1-12.

14. A controller unit configured to determine calibration values for use in deriving an estimated patient blood pressure measurement, based on measured pulse arrival time (PAT), the controller unit operably coupleable in use with a blood pressure measurement device, the controller configured to:

obtain one or more PAT values representative of a time difference between the start of a pre-ejection period of a heart cycle and the arrival of a corresponding blood pulse wave at a pre-determined first point on the body, as a result of blood flow from the heart to the first point through an arterial path of length, L;
receive from the blood pressure measurement device sensor data representative of at least one measured blood pressure value, Po, measured along a length portion $L_c$ of the arterial path, located between the heart and the pre-determined first point, wherein the blood pressure measurement device is configured to obtain blood pressure measurements through applying a varying external pressure, $P_{ext}$, across said length portion of the arterial path, $L_c$, wherein the one or more PAT values each correspond to PAT obtained at a different applied pressure, $P_{ext}$;

calculate, based on the one or more PAT values, and the corresponding $P_{ext}$ values, and on $P_0$, one or more coefficients, *m*, which relate a pulse wave velocity (PWV) along one or more sections of the arterial path with transmural pressure ($P_0$-$P_{ext}$) by modeling the one or more PAT values as a non-linear function of $P_{ext}$, wherein the non-linear function takes the form:

$$\mathrm{PAT}(P_{ext}) = (L\text{-}L_c)\mathbf{f}(P_0, m) + L_c\mathbf{f}(P_0\text{-}P_{ext}, m) + \mathrm{PEP}$$

where f(x, m) is an inverse function of x of the form f(x, m) $\propto$ 1/(mx + n), where n is a constant, and where PEP is a time duration of a pre-ejection period of the heart cycle.

15. A system for determining calibration values for use in deriving an estimated patient blood pressure measurement, based on measured pulse arrival time (PAT), the system comprising:

a controller unit according to claim 14; and
a blood pressure measurement device operatively coupled to the controller unit.

FIG. 1

FIG. 2

FIG. 3

FIG. 4

FIG. 5

Europäisches
Patentamt

European
Patent Office

Office européen
des brevets

# EUROPEAN SEARCH REPORT

Application Number

EP 19 19 3511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/029196 A1 (SOTERA WIRLESS INC [US]) 25 February 2016 (2016-02-25) | 1-7,9-15 | INV. A61B5/021 |
| Y | * equations 3,4,9,13 * * paragraph [0007] - paragraph [0015] * * paragraph [0039] - paragraph [0046] * * paragraph [0050] - paragraph [0053] * ----- | 8 | A61B5/022 A61B5/1495 A61B5/00 |
| Y | EP 3 430 991 A1 (KONINKLIJKE PHILIPS NV [NL]) 23 January 2019 (2019-01-23) | 8 | |
| A | * paragraph [0038] - paragraph [0042] * ----- | 10,11 | |
| A | US 2016/345844 A1 (MCCOMBIE DEVIN [US] ET AL) 1 December 2016 (2016-12-01) * paragraph [0112] - paragraph [0116] * ----- | 1,13,14 | |
| A | R. A. PAYNE ET AL: "Pulse transit time measured from the ECG: an unreliable marker of beat-to-beat blood pressure", JOURNAL OF APPLIED PHYSIOLOGY, vol. 100, no. 1, 1 September 2005 (2005-09-01), pages 136-141, XP055666210, US ISSN: 8750-7587, DOI: 10.1152/japplphysiol.00657.2005 * page 139, column 1, line 10 - column 2, line 2 * ----- | 1,13,14 | |

TECHNICAL FIELDS SEARCHED (IPC)

A61B

The present search report has been drawn up for all claims

| Place of search | Date of completion of the search | Examiner |
|---|---|---|
| The Hague | 11 February 2020 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 3511

11-02-2020

| Patent document cited in search report | | Publication date | Patent family member(s) | | Publication date |
|---|---|---|---|---|---|
| WO 2016029196 | A1 | 25-02-2016 | EP | 3182889 A1 | 28-06-2017 |
| | | | US | 2016143546 A1 | 26-05-2016 |
| | | | US | 2018303355 A1 | 25-10-2018 |
| | | | WO | 2016029196 A1 | 25-02-2016 |
| EP 3430991 | A1 | 23-01-2019 | EP | 3430991 A1 | 23-01-2019 |
| | | | WO | 2019016405 A1 | 24-01-2019 |
| US 2016345844 | A1 | 01-12-2016 | CN | 106028918 A | 12-10-2016 |
| | | | EP | 3102097 A1 | 14-12-2016 |
| | | | US | 2016345844 A1 | 01-12-2016 |
| | | | WO | 2015120330 A1 | 13-08-2015 |

EPO FORM P0459

For more details about this annex : see Official Journal of the European Patent Office, No. 12/82